# FASCICULE DE BREVET EUROPEEN

(11) **EP 2 294 155 B1**
(45) Date de publication et mention de la délivrance du brevet: **28.03.2012**
(21) Numéro de dépôt: 09761879.7
(22) Date de dépôt: 04.06.2009
(51) Int. Cl.: C09J 201/02, A61L 15/22, C09J 177/00, A61L 15/26, A61L 15/58

(54) **NOUVELLE COMPOSITION ADHESIVE THERMOFUSIBLE A BASE DE POLYAMIDE**
NEUE POLYAMIDBASIERTE SCHMELZKLEBERZUSAMMENSETZUNG
NOVEL POLYAMIDE-BASED HOT-MELT ADHESIVE COMPOSITION

(30) Priorité: 13.06.2008 FR 0803286
(43) Date de publication de la demande: 16.03.2011
(73) Titulaire: Bostik SA, 92400 Courbevoie (FR)
(72) Inventeur: SAJOT, Nicolas, F-60280 Clairoix (FR); BRUNET, Christille, F-75012 Paris (FR); LACHHAB, Majdouline, F-60170 Tracy Le Val (FR)
(74) Mandataire: Granet, Pierre
(86) Numéro de dépôt international: PCT/FR2009/000647
(87) Numéro de publication internationale: WO 2009/150328

(56) Documents cités:
- WO-A-03/059964
- WO-A-2008/029065
- BRUNSVELD L ET AL: "Supramolecular polymers" CHEMICAL REVIEWS, ACS,WASHINGTON, DC, US, vol. 101, 1 décembre 2001 (2001-12-01), pages 4071-4097, XP002267453 ISSN: 0009-2665
- PHILIPPE CORDIER ET AL: "Self-healing and thermoreversible rubber from supramolecular assembly" NATURE, NATURE PUBLISHING GROUP, LONDON, GB LNKD- DOI:10.1038/NATURE06669, vol. 451, 21 février 2008 (2008-02-21), pages 977-980, XP002576220 ISSN: 0028-0836

## Description

La présente invention a pour objet une composition adhésive thermofusible comprenant un polyamide et un polymère supramoléculaire dont les motifs comprennent un groupement de type imidazolidone. Elle concerne également l'utilisation de ladite composition pour la fabrication de produits d'hygiène jetables.

Les adhésifs (ou colles) thermofusibles (souvent désignés sous l'appellation anglaise de "Hot Melt adhesives" ou HM) sont des substances solides à température ambiante qui ne contiennent ni eau ni solvant. Ils sont appliqués à l'état fondu après chauffage à une température généralement comprise entre 80 et 250°C, le plus souvent entre 150 et 180°C et se solidifient lors du refroidissement, formant ainsi un joint qui assure la fixation des substrats à assembler. Ces adhésifs thermofusibles se présentent habituellement sous la forme de compositions qui comprennent un polymère thermoplastique et éventuellement une résine tackifiante et un plastifiant.

Les compositions adhésives thermofusibles sont utilisées dans l'industrie dans un large domaine d'applications comme par exemple la fabrication d'emballages rigides à base de papier et de carton, la confection des reliures dans l'édition, la réalisation d'assemblages divers dans les domaines de l'électronique, de l'automobile, du textile ou encore la fabrication d'articles d'hygiène jetables.

Ce dernier domaine concerne notamment les couches culottes jetables et les protections féminines ; les colles thermofusibles y sont utilisées pour la réalisation d'assemblages de feuilles minces et de substrats poreux de différents matériaux par contrecollage. On peut citer comme exemple de tels assemblages dans le cas des couches culottes le contrecollage d'une feuille de PolyEthylène (PE) avec une toile non tissée de PolyPropylène (PP), cette dernière offrant un aspect soyeux agréable au regard comme au toucher.

Le contrecollage de telles feuilles (ou substrats) est industriellement réalisé par un procédé qui comprend :
- le chauffage (à une température comprise entre 80 et 250°C, et de préférence entre 150 et 180 °C) de la composition adhésive thermofusible dans un bac appelé fondoir, jusqu'à obtention de sa fusion, puis
- l'enduction de l'un des 2 substrats à assembler, résultant dans le dépôt sur celui-ci, au moyen d'une buse, d'une couche de ladite composition à l'état fondu dont l'épaisseur est contrôlée et est en général comprise entre 1 et 500 µm, et enfin
- la mise en contact sous pression du substrat ainsi enduit avec le substrat à contrecoller ou à assembler.

Les dispositifs utilisés pour la mise en oeuvre de ces procédés de contrecollage sont généralement des machines qui fonctionnent en continu avec des vitesses de ligne souvent élevées et dans lesquelles par exemple tant les éléments à contrecoller ou assembler (feuilles, films ou autres substrats) que le produit final, souvent désigné par le terme de "complexe", sont en raison de leurs très grandes dimensions, conditionnés par enroulement sous forme de bobines de largeur et diamètre importants.

L'étape d'enduction par la composition adhésive thermofusible à l'état fondu comprend son passage à travers une ou plusieurs buses à une pression élevée de l'ordre de quelques bars à plus de 100 bars, de manière à obtenir un bon contact (ou mouillage) avec le substrat à enduire, lequel mouillage contribue à donner à l'assemblage final des 2 substrats le niveau de cohésion nécessaire. Ce dernier est habituellement quantifié par un test dit de pelage.

Un but de la présente invention est de proposer une composition adhésive thermofusible permettant d'obtenir pour l'assemblage (ou contrecollage) de 2 substrats un niveau de cohésion acceptable et/ou amélioré à la température d'utilisation du produit complexe, qui se situe en général dans un domaine de température proche de l'ambiante.

Il est également nécessaire que la composition adhésive thermofusible mise en oeuvre dans le procédé de contrecollage présente certaines caractéristiques physico-chimiques la rendant apte à cette mise en oeuvre.

Ainsi, ladite composition doit rester homogène à la température correspondant aux étapes du procédé, notamment depuis le fondoir jusqu'à la ou les buses d'enduction. Ce caractère homogène doit également se maintenir dans le temps, car les industriels qui mettent en oeuvre le procédé de contrecollage (comme par exemple les fabricants d'articles d'hygiène jetables) peuvent être amenés à stocker la composition à l'état fondu dans le bac fondoir durant un ou deux jours. Enfin, le bon déroulement de l'étape d'enduction impose que la viscosité de la colle soit comprise dans un certain domaine en général compris, pour une température voisine de 150°C, entre environ 200 et 30000 mPa.s, de préférence entre 1 et 15 Pa.s. La viscosité variant généralement en raison inverse de la température, il faut donc chauffer la colle dans le fondoir de manière à amener sa viscosité dans le domaine de viscosité requis par la buse.

Un autre but de la présente invention est de proposer une composition adhésive thermofusible qui soit homogène jusqu'à une température élevée, par exemple à une température supérieure à 130°C, de préférence égale à environ 180°C et dont le caractère homogène soit maintenu après stockage à cette température durant quelques jours, par exemple jusqu'à 3 jours.

Un autre but de la présente invention est de proposer une composition adhésive thermofusible présentant à température élevée, par exemple à environ 150°C, une viscosité réduite. Une telle réduction est très souhaitable dans la mesure où elle permet d'abaisser la température à laquelle il faut porter le fondoir, et de réaliser corrélativement une économie énergétique. Cette réduction permet également de faciliter le transport de la colle dans le circuit d'alimentation de la machine, depuis le fondoir jusqu'aux buses d'enduction. Elle permet enfin pour une température d'enduction fixée d'obtenir, grâce à la fluidité améliorée de la colle, un mouillage plus efficace du substrat, ce qui contribue à une meilleure cohésion de l'assemblage final.

Un autre but de la présente invention est de proposer une composition adhésive thermofusible permettant la mise en oeuvre de l'étape d'enduction du procédé de contrecollage à une température plus basse, offrant ainsi la possibilité de procéder à l'enduction de substrats sensibles à la chaleur.

Un autre but de la présente invention est de proposer une composition adhésive thermofusible qui procure une bonne cohésion de l'assemblage final à température ambiante, tout en présentant une viscosité réduite à température élevée, par exemple entre 150 et 180°C, qui facilite ainsi sa mise en oeuvre.

Il a à présent été trouvé que les buts précédents peuvent être atteints en totalité ou en partie au moyen de la composition adhésive thermofusible qui est l'objet de la présente invention.

L' invention concerne donc une composition adhésive thermofusible comprenant :
- de 20 à 95 % d'un polyamide, et
- de 5 à 80 % d'un polymère supramoléculaire obtenu par réaction entre la 1-(2-aminoéthyl)-2-imidazolidone (UDETA) et un mélange comprenant :
- de 51 à 100 % d'un ou plusieurs dimères d'acides gras identiques ou différents et/ou d'un ou plusieurs trimères d'acides gras identiques ou différents ; et
- de 0 à 49 % d'un ou plusieurs monomères d'acides gras identiques ou différents ;
les % indiqués pour représenter les teneurs en ingrédients de ladite composition et dudit mélange étant des % poids/poids.

L'invention concerne également l'utilisation de la composition adhésive thermofusible telle que définie précédemment pour le contrecollage de substrats en vue de la fabrication d'articles d'hygiène jetables.

Les pourcentages indiqués dans le présent texte pour représenter les teneurs en ingrédients d'une composition sont, en l'absence d'indications contraires, des % poids/poids.

L'expression "polymère supramoléculaire" désigne une structure chimique qui est constituée par les molécules d'un même composé reliées entre elles par des liaisons de type hydrogène. Cette expression dérive par analogie du terme "polymère" qui est classiquement utilisé pour désigner une structure chimique obtenue par l'établissement de liaisons chimiques covalentes entre une (dans le cas d'un homopolymère) ou plusieurs (dans le cas d'un copolymère) unités répétitives appelées "motifs". Par analogie, le composé constituant un polymère supramoléculaire est également désigné par le terme de "motif".

Le polymère supramoléculaire compris dans la composition selon l'invention peut dériver d'un ou plusieurs motifs dont la masse peut varier de 200 à 9000 g/mole, de préférence de 200 à 2000 g/mole.

Un tel polymère supramoléculaire est décrit dans les demandes internationales WO 03/059964 et WO 2008/029065 qui mentionnent simplement, parmi diverses utilisations, une utilisation comme additif dans les colles thermofusibles.

Il a à présent été trouvé que le choix d'un polymère thermoplastique spécifique, à savoir le polyamide, conduit -contrairement à d'autres polymères thermoplastiques d'utilisation courante dans les Hot Melt- à une composition adhésive thermofusible qui présente avantageusement à la fois un caractère homogène et une viscosité réduite à une température comprise entre 150 et 180°C, tout en offrant un bon niveau de cohésion -à température ambiante- de l'assemblage entre 2 substrats et/ou feuilles de matériaux, notamment entre des feuilles utilisées dans le domaine des articles d'hygiène jetables comme le PP non tissé et le PE.

Les polyamides compris dans la composition adhésive thermofusible selon l'invention sont susceptibles d'être obtenus par une réaction :
- de condensation d'un diacide choisi parmi les dimères d'acides gras, l'acide dodécanedioïque, l'acide sébacique, l'acide azélaïque, l'acide adipique, avec une diamine choisie parmi l'éthylènediamine, la pipérazine, l'hexaméthylènediamine, la diéthylènetriamine, la triéthylènetétramine, le dipipédidylpropane, et un polyoxypropylènediamine ; ou bien
- de polymérisation d'un aminoacide linéaire ou cyclique choisi parmi le caprolactame, le lauryl lactame ou l'acide 11-amino undécanoïque.

Les polyamides au sens de la présente invention incluent également les copolyamides susceptibles d'être obtenus par réaction d'un mélange de diacides avec un mélange de diamines, ainsi que les copolymères blocs comprenant un bloc polyamide susceptible d'être obtenu par les réactions de condensation ou de polymérisation définies précédemment.

On préfère utiliser comme diacide un dimère d'acide gras, l'acide adipique ou leur mélange, et comme diamine la pipérazine, l'éthylènediamine ou leur mélange.

Le polymère supramoléculaire utilisable dans la composition selon l'invention peut être préparé par les procédés décrits dans les demandes WO 03/059964 et WO 2008/029065.

Les acides gras dimères et trimères sont obtenus par polymérisation à haute température et sous pression d'acides gras insaturés, dits "monomères", qui comprennent de 6 à 22 atomes de carbones, de préférence de 12 à 20, et proviennent de sources végétales ou animales. On peut citer comme exemple de tels acides gras insaturés (monomères) les acides en C18 ayant une ou deux doubles liaisons (respectivement l'acide oléïque ou linoléïque) obtenus à partir de tallol qui est un sous-produit de la fabrication de la pâte à papier.

Après polymérisation de ces acides gras insaturés, on obtient un mélange technique contenant en moyenne 30-35 % d'acides monocarboxyliques (acides monomères) souvent isomérisés par rapport aux acides de départ, 60-65 % d'acides dicarboxyliques (acides dimères) avec le double de nombre de carbone par rapport aux acides de départ et 5-10 % d'acides tricarboxyliques (acides trimères) ayant le triple de nombre de carbone par rapport aux acides de départ. Par purification de ce mélange on obtient les différents grades commerciaux d'acides dimères, monomères ou trimères qui peuvent exister sous forme hydrogénée ou non hydrogénée. Parmi ceux-ci on peut citer la gamme Pripol^{®} développée par la Société Uniqema.

Selon un mode de réalisation plus particulièrement préféré, le polymère supramoléculaire est obtenu par une réaction, selon des quantités sensiblement stoechiométriques, de l'UDETA avec un mélange d'acides gras monomère et/ou dimère et/ou trimère issus de la polymérisation d'acides gras monomères insaturés majoritairement en C18, ledit mélange comprenant :
- de 1 à 3 % de monomères,
- de 75 à 80 % de dimères, et
- de 18 à 22 % de trimères.

Ce dernier mélange est disponible commercialement sous la dénomination de PRIPOL^{®} 1017 (Indice d'Acide égal à 193,4 mg KOH/g) de la Société Uniqema. Le polymère supramoléculaire correspondant à ce dernier mode de réalisation est désigné ci-après par le terme de SUPRA ; le motif correspondant a une masse molaire moyenne d'environ 900 g/mole. Il est fait référence pour sa préparation à la demande internationale WO 2008/029065 pré-citée et notamment à l'exemple 8.

Une composition adhésive thermofusible comprenant de 65 à 95 % de polyamide et de 5 à 35 % de polymère supramoléculaire est plus particulièrement préférée.

Outre le polyamide et le polymère supramoléculaire, la composition adhésive thermofusible selon l'invention comprend de 0 à 40 % d'au moins une résine tackifiante, un autre polymère, un plastifiant ou une huile, un anti-oxydant, un parfum, un pigment, un colorant ou une charge comme du carbonate de calcium, de la silice, une argile ou du talc.

La composition adhésive thermofusible selon l'invention est préparée par simple mélange de ses composants à l'état fondu à une température comprise entre 80 et 200 °C jusqu'à obtention d'un mélange homogène. Un simple mélangeage dans un pot avec un agitateur rotatif à faible vitesse est suffisant. D'autres dispositifs de mélange tels que mélangeurs, malaxeurs ou extrudeurs peuvent convenir et sont bien connus de l'homme du métier.

L'invention concerne également l'utilisation de la composition adhésive thermofusible telle que définie précédemment pour le contrecollage de substrats en vue de la fabrication d'articles d'hygiène jetables. Les substrats peuvent être constitués de divers matériaux choisis par exemple parmi une polyoléfine, un polyester, l'acide polyacrylique, le polychlorure de vinyle, le polystyrène, du cuir, du bois, de la cellulose, du carton, du papier, du béton, du verre, de la céramique. Des matériaux élastiques peuvent également être utilisés. Ces susbstrats peuvent se présenter sous la forme de feuilles, de films (par exemple des films polymères métallisés) de toiles, de fibres, de mousse, de plaques, de fils, de bandes ou de rubans.

Les articles d'hygiène jetables comprennent notamment les couches pour bébé, les articles d'hygiène féminine, les articles pour incontinence adulte. Tout matériau pour les substrats parmi ceux cités précédemment et toute forme de substrat peuvent être utilisés, en envisageant toutes sortes de combinaisons, l'adhésif aidant à lier au moins deux substrats ensemble.

La quantité de colle appliquée est généralement comprise entre 0,5 et 100 gramme par mètre carré de préférence entre 1,5 et 15 gramme par mètre carré.

Les exemples suivants sont donnés à titre purement illustratif de l'invention et ne sauraient être interprétés pour en limiter la portée.

On décrit à présent les méthodes de test auxquelles ont été soumises les compositions adhésives thermofusibles préparées.

### Test de stabilité à 177°C :

La stabilité à 177°C de la composition adhésive est évaluée par un test consistant à en placer un échantillon de 200 g durant 3 jours dans une étuve ventilée portée à 177°C et à observer chaque jour l'apparition éventuelle d'une séparation de phases.

### Viscosité Brookfield à 150°C :

La viscosité Brookfield est mesurée à 150°C selon la norme ASTM D3236-73.

### Test de pelage après 24 heures et 1 semaine :

Le niveau de cohésion de l'assemblage contrecollé est évalué par le test de pelage dont le principe consiste en la détermination de la force nécessaire à la séparation (ou pelage) de 2 substrats liés par la composition adhésive.

On utilise comme dispositif de contrecollage une machine opérant en continu à une vitesse de ligne d'environ 200 m/minute, commercialisée par la société Nordson sous la dénomination de Coater CTL 4400. Dans cette machine, la buse d'enduction est une buse à lèvre dont l'orifice rectangulaire a pour longueur 2,5 cm et pour hauteur 500 µm ; la mise en contact du premier substrat enduit de composition adhésive avec le deuxième substrat est réalisée au moyen de 2 rouleaux presseurs.

Les 2 substrats mis en oeuvre sont :
- un film de polyéthylène (PE) d'épaisseur 20 µm et de largeur 15 cm, et
- une toile non tissée de 10 g/m² de même largeur qui est constituée de fibres de polypropylène (PP) ayant pour diamètre 10 µm.

Ces deux substrats sont conditionnés en bobine de 15 cm de laize.

La composition adhésive à tester est chauffée dans le bac fondoir à 160°C, puis est enduite sur le film de polyéthylène, résultant dans le dépôt sur ce dernier d'une couche continue en forme de bande de 2,5 cm de large et d'épaisseur environ 5 µm, qui est disposée perpendiculairement à l'axe de la bobine et au centre de la laize dudit film. La quantité de composition adhésive appliquée dans la zone enduite en forme de bande est de 5 g/m². La toile non tissée de PP est contrecollée sur le film de PE ainsi enduit.

L'assemblage obtenu est laissé pendant 24 heures à température ambiante et à 50% d'humidité relative. On découpe alors la bande contrecollée de 2,5 cm de large correspondant à la zone enduite de manière à obtenir une éprouvette de forme rectangulaire d'environ 10 cm de longueur.

On décolle, à partir d'une extrémité de l'éprouvette de bande contrecollée et sur environ 2 cm, les 2 substrats individuels. Les 2 extrémités libres ainsi obtenues sont fixées sur deux dispositifs d' attache reliés, respectivement, à une partie fixe et une partie mobile d'un appareil de traction qui sont situées sur un axe vertical.

Alors qu'un mécanisme d'entraînement communique à la partie mobile une vitesse uniforme de 300 mm/minute, conduisant au décollement des 2 couches dont les extrémités décollées se déplacent progressivement selon un axe vertical en formant un angle de 180°, la partie fixe -reliée à un dynamomètre- mesure la force supportée par l'éprouvette ainsi maintenue.

Le résultat correspondant au pelage après 24 heures est exprimé en N/cm.

Le pelage après 1 semaine est mesuré de la même manière en laissant reposer l'assemblage obtenu après fabrication durant 1 semaine à température ambiante et 50% d'humidité relative.

### Polyamide PA :

On utilise dans les exemples un polyamide (ci-après désigné par PA) ayant une viscosité Brookfield à 205° C d'environ 5 Pa.s, qui est obtenu par réaction :
- d'un mélange de diacides comprenant en moles 85 % d'un dimère d'acide gras comprenant environ 18 atomes de carbone et 15 % d'acide adipique, avec
- un mélange de diamines comprenant en moles 90 % de pipérazine et 10 % d'éthylènediamine.

### Polymère supramoléculaire SUPRA :

Dans les exemples, on utilise comme polymère supramoléculaire le SUPRA tel que défini précédemment.

### Exemple 1 (Référence) : Composition adhésive thermofusible comprenant 100% de polyamide PA

Le test de stabilité à 177°C donne un résultat acceptable : aucun déphasage n'est noté après 3 jours.

La viscosité Brookfield à 150°C est de 26 Pa.s

Le résultat du test de pelage après 24 heures et 1 semaine est dans les 2 cas de 0,4 N/cm.

### Exemple 2 : Composition adhésive thermofusible comprenant 70% de polyamide PA et 30 % de SUPRA

On prépare par simple mélange au moyen d'un mélangeur tripale à chaud (température de 170°C) une composition homogène à l'état fondu comprenant 70% de polyamide PA et 30 % de SUPRA

Le test de stabilité à 177°C donne un résultat acceptable : aucun déphasage n'est noté après 3 jours.

La viscosité Brookfield à 150°C est de 4 Pa.s

Le résultat du test de pelage après 24 heures et 1 semaine est dans les 2 cas de 1 N/cm.

Cette composition présente ainsi par rapport à la référence une viscosité réduite à 150°C tout en offrant un niveau de cohésion plus que doublé à température ambiante.

### Exemple 3 : Composition adhésive thermofusible comprenant 90% de polyamide PA et 10 % de SUPRA:

On répète l'exemple 2 en préparant une composition comprenant 90% de polyamide PA et 10 % de SUPRA

On obtient également une composition homogène et stable selon le test à 177°C.

La viscosité Brookfield à 150°C est de 12 Pa.s

Le résultat du test de pelage après 24 heures et 1 semaine est dans les 2 cas de 0,7 N/cm.

### Exemple 4 (comparatif) : Composition adhésive thermofusible comprenant 70% d'EVA et 30 % de SUPRA :

On répète l'exemple 2 en remplaçant le polyamide PA par un copolymère d'éthylène et d'acétate de vinyle (EVA) obtenu à partir d'un mélange consistant de 33 % d'acétate de vinyle et 67 % d'éthylène, ayant un Melt Flow Index (MFI) de 45 g/10 minutes et disponible dans le commerce sous la dénomination Evatane 33-45 de la société Arkema.

On obtient également une composition homogène à l'état fondu.

Le test de stabilité à 177°C fait apparaître dès 1 jour une séparation de phase.

### Exemple 5 (comparatif) : Composition adhésive thermofusible comprenant 70% d'un copolymère d'éthylène et 30 % de SUPRA

On répète l'exemple 4 en utilisant un copolymère d'éthylène et d'octène ayant une densité de 0,870 qui est obtenu par catalyse métallocène à partir d'un mélange comprenant de l'éthylène et entre 1 et 10 % d'octène. Ce copolymère a un Melt Flow Index (MFI) de 1000 g/10 minutes et est disponible dans le commerce sous la dénomination Affinity GA 1900 de la société Dow Chemical.

On obtient les mêmes résultats.

### Exemple 6 (comparatif) : Composition adhésive thermofusible comprenant 70% d'un homopolymère de polypropylène (catalyse métallocène) et 30 % de SUPRA :

On répète l'exemple 4 en utilisant un homopolymère de polypropylène obtenu par catalyse métallocène qui est disponible dans le commerce sous la dénomination Licocène PP 1602 de la société Clariant.

On obtient les mêmes résultats.

### Exemple 7 (comparatif) : Composition adhésive thermofusible comprenant 70% de SIS et 30 % de SUPRA

On répète l'exemple 4 en utilisant un copolymère à bloc styrénique de type SIS (styrène-isoprène-styrène) ayant une teneur en styrène de 44 % et une teneur en dibloc SI de 0 %, qui est disponible dans le commerce sous la dénomination Vector 4411 de la société Dexco Polymers.

Le SIS et le SUPRA sont incompatibles et ne permettent pas la préparation d'un mélange homogène.

## Revendications

1. Composition adhésive thermofusible comprenant :
- de 20 à 95 % d'un polyamide, et
- de 5 à 80 % d'un polymère supramoléculaire obtenu par réaction entre la 1-(2-aminoéthyl)-2-imidazolidone (UDETA) et un mélange comprenant :
- de 51 à 100 % d'un ou plusieurs dimères d'acides gras identiques ou différents et/ou d'un ou plusieurs trimères d'acides gras identiques ou différents ; et
- de 0 à 49 % d'un ou plusieurs monomères d'acides gras identiques ou différents ;
les % indiqués pour représenter les teneurs en ingrédients de ladite composition et dudit mélange étant des % poids/poids.

2. Composition adhésive thermofusible selon la revendication 1, **caractérisée en ce que** le polyamide est susceptible d'être obtenu par une réaction :
- de condensation d'un diacide choisi parmi les dimères d'acides gras, l'acide dodécanedioïque, l'acide sébacique, l'acide azélaïque, l'acide adipique, avec une diamine choisie parmi l'éthylènediamine, la pipérazine, l'hexaméthylènediamine, la diéthylènetriamine, la triéthylènetétramine, le dipipéridylpropane, et un polyoxypropylènediamine ; ou bien
- de polymérisation d'un aminoacide linéaire ou cyclique choisi parmi le caprolactame, le lauryl lactame ou l'acide 11-amino undécanoïque.

3. Composition adhésive thermofusible selon la revendication 2, **caractérisée en ce que** l'on utilise comme diacide un dimère d'acide gras, l'acide adipique ou leur mélange, et comme diamine la pipérazine, l'éthylènediamine ou leur mélange.

4. Composition adhésive thermofusible selon l'une des revendications 1 à 3, **caractérisée en ce que** le polymère supramoléculaire est obtenu par une réaction, selon des quantités sensiblement stoechiométriques, de l'UDETA avec un mélange d'acides gras monomère et/ou dimère et/ou trimère issus de la polymérisation d'acides gras monomères insaturés majoritairement en C18, ledit mélange comprenant :
- de 1 à 3 % de monomères,
- de 75 à 80 % de dimères, et
- de 18 à 22 % de trimères.

5. Composition adhésive thermofusible selon l'une des revendications 1 à 4, **caractérisée en ce qu'**elle comprend de 65 à 95 % de polyamide et de 5 à 35 % de polymère supramoléculaire.

6. Utilisation de la composition adhésive thermofusible telle que définie dans l'une des revendications 1 à 5 pour le contrecollage de substrats en vue de la fabrication d'articles d'hygiène jetables.

## Claims

1. Hot-melt adhesive composition comprising:
- from 20 to 95% of a polyamide, and
- from 5 to 80% of a supramolecular polymer obtained by reaction between 1-(2-aminoethyl)-2-imidazolidone (UDETA) and a mixture comprising:
- from 51 to 100% of one or more identical or different fatty acid dimers and/or of one or more identical or different fatty acid trimers; and
- from 0 to 49% of one or more identical or different fatty acid monomers;
the % indicated to represent the contents of ingredients of the said composition and the said mixture being % weight/weight.

2. Hot-melt adhesive composition according to Claim 1, **characterized in that** the polyamide is capable of being obtained by:
- a condensation reaction of a diacid chosen from fatty acid dimers, dodecanedioic acid, sebacic acid, azelaic acid and adipic acid with a diamine chosen from ethylenediamine, piperazine, hexamethylenediamine, diethylenetriamine, triethylenetetramine, dipiperidylpropane and a polyoxypropylenediamine; or else
- a polymerization reaction of a linear or cyclic amino acid chosen from caprolactam, lauryllactam or 11-aminoundecanoic acid.

3. Hot-melt adhesive composition according to Claim 2, **characterized in that** use is made, as diacid, of a fatty acid dimer, adipic acid or their mixture and, as diamine, of piperazine, ethylenediamine or their mixture.

4. Hot-melt adhesive composition according to one of Claims 1 to 3, **characterized in that** the supramolecular polymer is capable of being obtained by a reaction, according to substantially stoichiometric amounts, of UDETA with a mixture of monomeric and/or dimeric and/or trimeric fatty acids resulting from the polymerization of predominantly C₁₈ unsaturated monomeric fatty acids, said mixture comprising:
- from 1 to 3% of monomers,
- from 75 to 80% of dimers, and
- from 18 to 22% of trimers.

5. Hot-melt adhesive composition according to one of Claims 1 to 4, **characterized in that** it comprises from 65 to 95% of polyamide and from 5 to 35% of supramolecular polymer.

6. Use of the hot-melt adhesive composition as defined in one of Claims 1 to 5 in the laminating of substrates for the purpose of the manufacture of disposable hygiene articles.

## Patentansprüche

1. Schmelzklebstoffzusammensetzung, umfassend:
- 20 bis 95% eines Polyamids und
- 5 bis 80% eines durch Reaktion zwischen 1-(2-Aminoethyl)-2-imidazolidon (UDETA) und einer Mischung, umfassend:
- 51 bis 100% eines oder mehrerer gleicher oder verschiedener Fettsäuredimere und/oder eines oder mehrerer gleicher oder verschiedener Fettsäuretrimere und
- 0 bis 49% eines oder mehrerer gleicher oder verschiedener Fettsäuremonomere,
erhaltenen supramolekularen Polymers, wobei sich die Prozentangaben zur Wiedergabe der Gehalte von Bestandteilen der Zusammensetzung und der Mischung auf das Gewicht beziehen.

2. Schmelzklebstoffzusammensetzung nach Anspruch 1, **dadurch gekennzeichnet, dass** das Polyamid durch
- eine Kondensationsreaktion einer Disäure, die unter Fettsäuredimeren, Dodecandisäure, Sebacinsäure, Azelainsäure und Adipinsäure ausgewählt ist, mit einem Diamin, das unter Ethylendiamin, Piperazin, Hexamethylendiamin, Diethylentriamin, Triethylentetramin, Dipiperidylpropan und einem Polyoxypropylendiamin ausgewählt ist; oder auch
- eine Polymerisationsreaktion einer linearen oder cyclischen Aminosäure, die unter Caprolactam, Lauryllactam oder 11-Aminoundecansäure ausgewählt ist;
erhältlich ist.

3. Schmelzklebstoffzusammensetzung nach Anspruch 2, **dadurch gekennzeichnet, dass** man als Disäure ein Fettsäuredimer, Adipinsäure oder eine Mischung davon und als Diamin Piperazin, Ethylendiamin oder eine Mischung davon verwendet.

4. Schmelzklebstoffzusammensetzung nach einem der Ansprüche 1 bis 3, **dadurch gekennzeichnet, dass** das supramolekulare Polymer durch eine Reaktion von UDETA mit einer Mischung von monomeren und/oder dimeren und/oder trimeren Fettsäuren aus der Polymerisation von ungesättigten monomeren Fettsäuren, die überwiegend 18 C-Atome aufweisen, wobei die Mischung:
- 1 bis 3% Monomere,
- 75 bis 80% Dimere und
- 18 bis 22% Trimere
umfasst, in weitgehend stöchiometrischen Mengen erhalten wird.

5. Schmelzklebstoffzusammensetzung nach einem der Ansprüche 1 bis 4, **dadurch gekennzeichnet, dass** sie 65 bis 95% Polyamid und 5 bis 35% supramolekulares Polymer umfasst.

6. Verwendung der Schmelzklebstoffzusammensetzung gemäß einem der Ansprüche 1 bis 5 zum Kaschieren von Substraten zur Herstellung von Wegwerfhygieneartikeln.
